(19) Europäisches Patentamt
European Patent Office
Office européen des brevets

(11) EP 3 016 631 B1

(12) **EUROPEAN PATENT SPECIFICATION**

(45) Date of publication and mention of the grant of the patent:
**02.10.2019 Bulletin 2019/40**

(21) Application number: **14731393.6**

(22) Date of filing: **21.05.2014**

(51) Int Cl.:
***A61K 8/36*** (2006.01) ***A61Q 17/04*** (2006.01)
***A61K 8/81*** (2006.01) ***A61K 8/02*** (2006.01)

(86) International application number:
**PCT/JP2014/064044**

(87) International publication number:
**WO 2014/192780 (04.12.2014 Gazette 2014/49)**

(54) **COSMETIC COMPOSITION**
KOSMETISCHE ZUSAMMENSETZUNG
COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.05.2013 JP 2013113792**

(43) Date of publication of application:
**11.05.2016 Bulletin 2016/19**

(73) Proprietors:
- **L'OREAL**
  **75008 Paris (FR)**
  Designated Contracting States:
  **AT BE BG CH CY CZ DE DK EE ES FI FR GB GR HR HU IE IS IT LI LT LU LV MC MK MT NL NO PL PT RO RS SE SI SK SM TR**
- **Amazaki, Kyoko**
  **Tokyo, 134-0043 (JP)**
  Designated Contracting States:
  **AL**

(72) Inventor: **AMAZAKI, Kyoko**
**Tokyo, 134-0043 (JP)**

(74) Representative: **Casalonga**
**Casalonga & Partners**
**Bayerstraße 71/73**
**80335 München (DE)**

(56) References cited:
**EP-A2- 2 011 481**
**WO-A1-2010/043588**
**WO-A1-2011/042358**
**US-A1- 2005 031 655**
**US-B1- 6 495 123**

- **DATABASE GNPD [Online] MINTEL; April 2013 (2013-04), "Dry-Touch Gel-Cream SPF 50+", XP002731567, Database accession no. 2028476**
- **DATABASE GNPD [Online] MINTEL; February 2013 (2013-02), "Dry Touch Facial Daily Sun Protection SPF 30", XP002731568, Database accession no. 1998588**
- **Anonymous: "Personal Care SUNSPHERES(TM) Hollow Sphere Technology An SPF Booster for More Aesthetically Pleasing Formulations Features, Benefits and Applications", , 28 February 2006 (2006-02-28), pages 1-14, XP055321502, Retrieved from the Internet: URL:http://www.dow.com/assets/attachments/business/pcare/sunspheres/sunspheres_powder/tds/sunspheres_powder.pdf [retrieved on 2016-11-22]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a cosmetic composition in the form of an O/W emulsion comprising an aqueous phase and a fatty phase, as well as specific selected ingredients, and also relates to a non-therapeutic cosmetic method using the same.

### BACKGROUND ART

Aging skin is the result of more than just chronological age. Skin is exposed to various environmental stresses, such as UV rays, which cause free radicals to form in the skin. Free radicals include, for example, singlet oxygen, hydroxyl radicals, the superoxide anion, nitric oxide, and hydrogen radicals. The end result is a loss of elasticity of the skin and the appearance of wrinkles leading to premature ageing of the skin. This process is commonly referred to as photo-aging.

Generally, UV sunscreen actives are utilized to provide protection from UV light. Numerous cosmetic compositions intended for the photoprotection (UV-A and/or UV-B) of the skin have been proposed to date. Examples are among other described in US 2005/0031655 A1, US 6,495,123 B1, WO 2011/042358 A1 or the Internet publication "Personal Care SUNSPHERES™ Hollow Sphere Technology An SPF Booster for More Aesthetically Pleasing Formulations Features, Benefits and Applications" (http:// www.dow.com/assets/attachments/business/pcare/sunspheres/sunspheres_powder/tds/sunspheres_powder.pdf).

These anti-sun compositions often take the form of oil-in-water or water-in-oil emulsions, of gels, or of non-aqueous products which contain, in various concentrations, one or more insoluble and/or fat-soluble and/or water-soluble, organic and/or inorganic screening agents that are capable of selectively absorbing the harmful UV radiation. These screening agents and their amounts are selected as a function of the desired protection index. Depending on their lipophilic or, alternatively, hydrophilic character, these screening agents may become distributed, respectively, in either the fatty phase or the aqueous phase of the final composition.

It is possible to enhance the UV shielding ability of a UV protection product by increasing the amount of UV filter(s) in the product. However, if the UV filter is lipophilic, a large amount of the lipophilic UV filter(s) can cause an undesirable oily and heavy texture.

Thus, there has been a need for a UV protection product with both high UV-shielding ability and a fresh, water-like texture, because it can provide effective UV protection and good feeling during use.

### DISCLOSURE OF INVENTION

One of the objectives of the present invention is to provide a cosmetic composition for UV shielding which can provide high UV-shielding ability, while providing a fresh water-like texture, thereby realizing effective UV protection and good feeling during use.

The above objective of the present invention can be attained by a cosmetic composition in the form of an O/W emulsion, comprising, in a cosmetically acceptable medium:

(a) at least one aqueous phase comprising water;
(b) at least one fatty phase comprising,

  (i) at least one polar oil,
  (ii) at least one lipophilic UV filter, and
  (iii) at least one fatty acid;

(c) at least one anionic associative polymer; and
(d) at least one hollow particle.

It is preferable that the amount of water in the composition according to the present invention be equal to or more than 55% by weight in relation to the total weight of the composition.

It is preferable that the (b)-(i) polar oil be selected from the group consisting of $C_{12}$-$C_{15}$ alcohol benzoate, diisopropyl sebacate, isopropyl lauroyl sarcosinate, dicaprylyl carbonate, 2-phenylethyl benzoate, butyloctyl salicylate, 2-octyldodecyl neopentanoate, dicaprylyl ether, isocetyl stearate, isodecyl neopentanoate, isononyl isononate, isopropyl myristate, isopropyl palmitate, isostearyl behenate, myristyl myristate, octyl palmitate, and tridecyl trimellitate. It is preferable that the (b)-(ii) lipophilic UV filter be selected from the group consisting of butylmethoxydibenzoylmethane, ethylhexyl methoxycinnamate, ethylhexyl salicylate, homosalate, butylmethoxydibenzoylmethane, octocrylene, benzophenone-3, n-hexyl 2-(4-diethylamino-2-hydroxybenzoyl)benzoate, 4-methylbenzylidenecamphor, bis(ethylhexyloxyphenyl)methoxyphenyltriazine, ethylhexyl triazone, diethylhexyl butamido triazone, 2,4,6-tris(dineopentyl 4'-aminobenzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4,6-tris(biphenyl-4-yl)-1,3,5-triazine, 2,4,6-tris(terphenyl)-1,3,5-triazine, drometrizole trisiloxane, polysilicone-15, 1,1-dicarboxy(2,2'-dimethylpropyl)-4,4-diphenylbutadiene, 2,4-bis[4-[5-(1,1-dimethylpropyl)benzoxazol-2-yl]phenylimino]-6-[(2-ethylhexyl)imino]-1,3, 5-triazine, and mixtures thereof.

It is preferable that the (b)-(iii) fatty acid be a linear or branched fatty acid having a carbon chain length of $C_{10-30}$.

It is preferable that the (c) associative polymer be selected from polymers comprising at least one hydrophilic unit of unsaturated olefinic carboxylic acid type, and at least one hydrophobic unit of a type such as a ($C_{10}$-$C_{30}$) alkyl ester of an unsaturated carboxylic acid.

It is preferable that the (d) hollow particle be made from a copolymer of styrene and (meth)acrylic acid or one of its $C_1$-$C_{20}$ alkyl esters.

The amount of the (b) fatty phase in the composition according to the present invention may be from 5% to 30% by weight, more preferably from 7% to 25% by weight, and even more preferably from 10% to 20% by weight in relation to the total weight of the composition.

The amount of the (b)-(i) polar oil in the composition according to the present invention may be from 1% to 15% by weight in relation to the total weight of the composition.

The amount of the (b)-(ii) lipophilic UV filter in the composition according to the present invention may be from 3% to 20% by weight in relation to the total weight of the composition.

The amount of the (b)-(iii) fatty acid in the composition according to the present invention may be from 0.1% to 5% by weight in relation to the total weight of the composition.

The amount of the (c) anionic associative polymer in the composition according to the present invention may be from 0.01% to 5% by weight in relation to the total weight of the composition.

The amount of the (d) hollow particle in the composition according to the present invention may be from 0.1% to 5% by weight in relation to the total weight of the composition.

The present invention also relates to a non-therapeutic cosmetic method for caring for and/or making up the skin, comprising applying the cosmetic composition according to the present invention.

The present invention also relates to a non-therapeutic cosmetic method for forming a substantially even film on the skin, comprising applying the cosmetic composition according to the present invention.

## BEST MODE FOR CARRYING OUT THE INVENTION

After diligent research, the inventors have discovered that it is possible to achieve the above objective by providing a cosmetic composition for protecting the skin, in particular the face, from UV rays, which is in the form of an O/W emulsion, where the cosmetic composition includes some specific selected ingredients.

Thus, the present invention relates to a cosmetic composition in the form of an O/W emulsion, comprising, in a cosmetically acceptable medium:

- (a) at least one aqueous phase comprising water;
- (b) at least one fatty phase comprising,
  - (i) at least one polar oil,
  - (ii) at least one lipophilic UV filter, and
  - (iii) at least one fatty acid;
- (c) at least one anionic associative polymer; and
- (d) at least one hollow particle.

The cosmetic composition according to the present invention has high UV-shielding ability, while providing a fresh, water-like texture, and therefore, it can simultaneously provide both effective UV protection and good feeling to use.

Furthermore, the cosmetic composition according to the present invention can form a homogeneous or even film on the skin to block UV rays penetrating into the skin, and therefore, it can provide even and uniform UV protection effects.

Hereinafter, the cosmetic composition according to the present invention will be explained in a more detailed manner.

[Cosmetic Composition]

The cosmetic composition according to the present invention is in the form of an O/W emulsion, and includes in a cosmetically acceptable medium:

(a) at least one aqueous phase comprising water;
(b) at least one fatty phase comprising,

(i) at least one polar oil,
(ii) at least one lipophilic UV filter, and
(iii) at least one fatty acid;

(c) at least one anionic associative polymer; and
(d) at least one hollow particle.

The "cosmetically acceptable medium" here means a medium which is not toxic and can be applied to the skin, lips, hair, scalp, lashes, brows, nails, or any other cutaneous region of the body, and can be rephrased as "physiologically acceptable medium".

The term "O/W emulsion" or "oil-in-water emulsion" means any macroscopically homogeneous composition comprising a continuous aqueous phase and a fatty phase dispersed in the said aqueous phase in the form of droplets.

Each of the components (a) to (d) will be explained below.

{Aqueous Phase}

The cosmetic composition according to the present invention includes at least one aqueous phase. Since the cosmetic composition according to the present invention is in the form of an O/W emulsion, the aqueous phase in the cosmetic composition according to the present invention can be the continuous phase of the O/W emulsion.

The aqueous phase includes water. The amount of water may be equal to or more than 55% by weight, preferably 60% by weight or more, more preferably 70% by weight or more, and even more preferably 80% by weight or more, in relation to the total weight of the composition. For example, the amount of water may be 55% to 90% by weight, preferably 65% to 90% by weight, more preferably 75% to 85% by weight, and even more preferably 80% to 85% by weight, in relation to the total weight of the composition.

The aqueous phase may include at least one polyol that is miscible with water at room temperature (25°C) chosen especially from polyols especially containing from 2 to 20 carbon atoms, preferably containing from 2 to 10 carbon atoms, and preferentially containing from 2 to 6 carbon atoms, such as glycerol, propylene glycol, butylene glycol, pentylene glycol, hexylene glycol, dipropylene glycol, or diethylene glycol; glycol ethers (especially containing from 3 to 16 carbon atoms) such as mono-, di-, or tripropylene glycol ($C_1$-$C_4$)alkyl ethers, mono-, di-, or triethylene glycol ($C_1$-$C_4$)alkyl ethers; and mixtures thereof. Such a polyol or polyols may promote the moisturization of the surface of the skin on which the composition is applied.

The aqueous phase may include at least one monoalcohol that is miscible with water at room temperature (25°C) chosen especially from monoalcohols containing from 2 to 10 carbon atoms, preferably containing from 2 to 6 carbon atoms, such as ethanol and isopropanol.

The pH of the aqueous phase is not limited, but is generally between about 3 and 12, preferably between about 5 and 11, and even more particularly from 6 to 8.5.

The pH of the aqueous phase may be controlled by the presence or absence of at least one acidifying agent and/or at least one basifying agent.

Among the acidifying agents that may be mentioned, for example, are mineral or organic acids, for instance hydrochloric acid, orthophosphoric acid, or sulfuric acid; carboxylic acids, for instance acetic acid, tartaric acid, citric acid, and lactic acid; and sulfonic acids.

Among the basifying agents, examples that may be mentioned include aqueous ammonia, alkali metal carbonates, alkanolamines such as mono-, di-, and triethanolamines and derivatives thereof, sodium hydroxide, potassium hydroxide, and the compounds of the formula below:

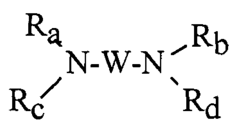

in which W is a propylene residue optionally substituted with a hydroxyl group or a $C_1$-$C_4$ alkyl radical; and $R_a$, $R_b$, $R_c$, and $R_d$, which may be identical or different, represent a hydrogen atom, or a $C_1$-$C_4$ alkyl or $C_1$-$C_4$ hydroxyalkyl radical.

The amount of the aqueous phase may be equal to or more than 60% by weight, preferably 70% by weight or more, more preferably 80% by weight or more, and even more preferably 85% by weight or more, in relation to the total weight of the composition. For example, the amount of the aqueous phase may be 70% to 95% by weight, preferably 75% to 93% by weight, more preferably 80% to 90% by weight, and even more preferably 85% to 90% by weight, in relation to the total weight of the composition.

{Fatty Phase}

The cosmetic composition according to the present invention includes at least one fatty phase. Since the cosmetic composition according to the present invention is in the form of an O/W emulsion, the fatty phase in the cosmetic composition according to the present invention can be the dispersed phases of the O/W emulsion.

The amount of the fatty phase may be equal to or less than 40% by weight, preferably 30% by weight or less, more preferably 20% by weight or less, and even more preferably 15% by weight or less, in relation to the total weight of the composition. For example, the amount of the fatty phase may be 5% to 30% by weight, preferably 7% to 25% by weight, more preferably 10% to 20% by weight, and even more preferably 10% to 15% by weight, in relation to the total weight of the composition.

(Polar Oil)

The fatty phase includes at least one polar oil. If two or more polar oils are used, they may be the same or different.

The term "polar oil" means any lipophilic compound having, at 25°C, a solubility parameter $\delta_d$ characteristic of dispersive interactions of greater than 16 and a solubility parameter $\delta_p$ characteristic of polar interactions strictly greater than 0. The solubility parameters $\delta_d$ and $\delta_p$ are defined according to the Hansen classification. For example, these polar oils may be chosen from esters, triglycerides, and ethers.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:

- $\delta_d$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{1/2}$. The parameters $\delta_p$, $\delta_h$, $\delta_d$, and $\delta_a$ are expressed in $(J/cm^3)^{1/2}$.

The polar oil may be a volatile or non-volatile hydrocarbon-based oil.

These oils may be of plant, mineral, or synthetic origin.

The term "polar hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine, and/or amide groups.

Preferentially, the polar oil according to the invention has a surface tension of greater than 10 mN/m at 25°C and atmospheric pressure.

The surface activity is measured by static tensiometry using the Du Noüy ring.

The principle of the measurement is as follows (measurement carried out at 25°C and atmospheric pressure): The weight of the ring is neutralized by a tare. The ring is completely immersed in the liquid to be evaluated, then withdrawn very slowly until the force reaches its maximum. From this maximum force $F_{max}$, the surface tension is calculated according to the equation:

$$\sigma = F_{max} / 4\pi R \, f_{corr} (r, R, \rho)$$

with $f_{corr}$: correction factor of the ring depending on the geometry of the ring and the density p.

The parameters r and R respectively denote the internal and external radii of the ring.

According to a first embodiment, the polar oil may be a non-volatile oil. In particular, the non-volatile polar oil may be chosen from the list of oils below, and mixtures thereof:

- hydrocarbon-based polar oils such as phytostearyl esters, such as phytostearyl oleate, phytostearyl isostearate, and lauroyl/octyldodecyl/phytostearyl glutamate (Ajinomoto, Eldew PS203), triglycerides consisting of fatty acid esters of glycerol, in particular the fatty acids of which may have chain lengths ranging from $C_4$ to $C_{36}$, and especially from $C_{18}$ to $C_{36}$, these oils possibly being linear or branched, and saturated or unsaturated; these oils may especially be heptanoic or octanoic triglycerides, wheat germ oil, sunflower oil, grape seed oil, sesame seed oil (820.6 g/mol), corn oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkin oil, marrow oil, blackcurrant oil, evening primrose oil, millet oil, barley oil, quinoa oil, rye oil, safflower oil, candlenut oil, passion flower oil, or musk rose oil; shea butter; or alternatively caprylic/capric acid triglycerides, for instance those sold by the company Stéarineries Dubois or those sold under the names Miglyol 810®, 812® and 818® by the company Dynamit Nobel;
- synthetic ethers containing from 10 to 40 carbon atoms, such as dicaprylyl ether;
- hydrocarbon-based esters of formula RCOOR' in which RCOO represents a carboxylic acid residue comprising from 2 to 40 carbon atoms, and R' represents a hydrocarbon-based chain containing from 1 to 40 carbon atoms, such as cetostearyl octanoate, isopropyl alcohol esters, such as isopropyl myristate or isopropyl palmitate, ethyl palmitate, 2-ethylhexyl palmitate, isopropyl stearate or isostearate, isostearyl isostearate, octyl stearate, diisopropyl adipate, heptanoates, and especially isostearyl heptanoate, alcohol or polyalcohol octanoates, decanoates or ricinoleates, for instance propylene glycol dioctanoate, cetyl octanoate, tridecyl octanoate, 2-ethylhexyl 4-diheptanoate, and palmitate, alkyl benzoate, polyethylene glycol diheptanoate, propylene glycol 2-diethyl hexanoate, and mixtures thereof, $C_{12}$ to $C_{15}$ alcohol benzoates, hexyl laurate, neopentanoic acid esters, for instance isodecyl neopentanoate, isotridecyl neopentanoate, isostearyl neopentanoate, and 2-octyldodecyl neopentanoate, isononanoic acid esters, for instance isononyl isononanoate, isotridecyl isononanoate, and octyl isononanoate, oleyl erucate, isopropyl lauroyl sarcosinate, diisopropyl sebacate, isocetyl stearate, isodecyl neopentanoate, isostearyl behenate, and myristyl myristate;
- polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: dilinoleic acid/butanediol copolymer), or else copolymers of polyols and of dimer diacids, and esters thereof, such as Hailuscent ISDA;
- polyol esters and pentaerythritol esters, for instance dipentaerythrityl tetrahydroxystearate/tetraisostearate;
- fatty alcohols containing from 12 to 26 carbon atoms, for instance octyldodecanol, 2-butyloctanol, 2-hexyldecanol, 2-undecylpentadecanol, and oleyl alcohol;
- $C_{12}$-$C_{22}$ higher fatty acids, such as oleic acid, linoleic acid, and linolenic acid, and mixtures thereof;
- fluorinated oils which are optionally partially hydrocarbon-based and/or silicone-based;
- fatty acids containing from 12 to 26 carbon atoms, for instance oleic acid;
- dialkyl carbonates, the two alkyl chains possibly being identical or different, such as dicaprylyl carbonate sold under the name Cetiol CC® by Cognis; and
- non-volatile oils of high molecular mass, for example between 400 and 10 000 g/mol, in particular between 650 and 10 000 g/mol, for instance:

    i) vinylpyrrolidone copolymers such as the vinylpyrrolidone/1-hexadecene copolymer, Antaron V-216 sold or manufactured by the company ISP (MW = 7300 g/mol),
    ii) esters such as:

        a) linear fatty acid esters with a total carbon number ranging from 35 to 70, for instance pentaerythrityl tetrapelargonate (MW = 697.05 g/mol),
        b) hydroxylated esters such as polyglycerol-2 triisostearate (MW = 965.58 g/mol),
        c) aromatic esters such as tridecyl trimellitate (MW = 757.19 g/mol), $C_{12}$-$C_{15}$ alcohol benzoate, 2-phenylethyl benzoate, and butyloctyl salicylate,
        d) esters of $C_{24}$-$C_{28}$ branched fatty acids or fatty alcohols such as those described in patent application EP-A-0 955 039, and especially triisoarachidyl citrate (MW = 1033.76 g/mol), pentaerythrityl tetraisononanoate (MW = 697.05 g/mol), glyceryl triisostearate (MW = 891.51 g/mol), glyceryl tris(2-decyl)tetradecanoate (MW = 1143.98 g/mol), pentaerythrityl tetraisostearate (MW = 1202.02 g/mol), polyglyceryl-2 tetraisostearate (MW = 1232.04 g/mol) or else pentaerythrityl tetrakis(2-decyl)tetradecanoate (MW = 1538.66 g/mol),
        e) esters and polyesters of dimer diol and of monocarboxylic or dicarboxylic acid, such as esters of dimer diol and of fatty acid and esters of dimer diol and of dimer dicarboxylic acid, such as Lusplan DD-DA5® and Lusplan DD-DA7® sold by the company Nippon Fine Chemical and described in patent application US 2004-175 338, the content of which is incorporated into the present application by reference,

- and mixtures thereof.

Preferably the polar oil is chosen from $C_{12}$-$C_{15}$ alcohol benzoate, diisopropyl sebacate, isopropyl lauroyl sarcosinate, dicaprylyl carbonate, 2-phenylethyl benzoate, butyloctyl salicylate, 2-octyldodecyl neopentanoate, dicaprylyl ether, isocetyl stearate, isodecyl neopentanoate, isononyl isononate, isopropyl myristate, isopropyl palmitate, isostearyl behenate, myristyl myristate, octyl palmitate, and tridecyl trimellitate.

Preferably, the polar oil is a $C_{12}$-$C_{15}$ alkyl benzoate or isopropyl lauroyl sarcosinate.

The amount of polar oil (excluding lipophilic UV filter(s)) may range from 1% to 15% by weight, preferably 2 to 10% by weight, and more preferably from 3% to 8% by weight, relative to the total weight of the composition.

The "fatty phase" of the compositions according to the invention may also comprise a wax, an apolar oil, or mixtures thereof.

The term "wax" is understood to mean a compound which is solid or substantially solid at room temperature and which has a melting point generally of greater than 35°C.

The apolar oils and the waxes conventionally used in cosmetic compositions may be used in the compositions according to the present invention.

(Lipophilic UV filter)

The fatty phase includes at least one lipophilic UV filter. If two or more lipophilic UV filters are used, they may be the same or different.

The term "lipophilic UV filter" means an organic molecule that is capable of screening out UV radiation between 290 and 400 nm, and which can be dissolved in a molecular form or dispersed in a fatty phase in order to obtain a macroscopically homogeneous phase. The term "organic molecule" means any molecule comprising in its structure one or more carbon atoms. Thus, the lipophilic UV filter used for the present invention may be active in the UV-A and/or UV-B region.

The lipophilic UV filter may be solid or liquid. The terms "solid" and "liquid" mean solid and liquid, respectively, at 25°C under 1 atm.

The lipophilic UV filter may be chosen especially from cinnamic derivatives; anthranilates; salicylic derivatives; dibenzoylmethane derivatives, camphor derivatives; benzophenone derivatives; β, β-diphenylacrylate derivatives; triazine derivatives; benzotriazole derivatives; benzalmalonate derivatives, especially those mentioned in patent US 5 624 663; imidazolines; p-aminobenzoic acid (PABA) derivatives; benzoxazole derivatives as described in patent applications EP 0 832 642, EP 1 027 883, EP 1 300 137, and DE 101 62 844; screening polymers and screening silicones such as those described especially in patent application WO 93/04665; α-alkylstyrene-based dimers, such as those described in patent application DE 198 55 649; 4,4-diarylbutadienes such as those described in patent applications EP 0 967 200, DE 197 46 654, DE 197 55 649, EP-A-1 008 586, EP 1 133 980, and EP 133 981; merocyanin derivatives such as those described in patent applications WO 04/006 878, WO 05/058 269, WO 06/032 741, FR 2 957 249, and FR 2 957 250; and mixtures thereof.

As examples of the lipophilic UV filter, mention may be made of those denoted hereinbelow under their INCI name:

Dibenzoylmethane derivative:
Butylmethoxydibenzoylmethane or avobenzone sold under the trade name Parsol 1789 by the company DSM Nutritional Products,

Para-aminobenzoic acid derivatives:

Ethyl PABA,
Ethyl Dihydroxypropyl PABA,
Ethylhexyl Dimethyl PABA sold in particular under the name Escalol 507 by ISP,

Salicylic derivatives:

Homosalate sold under the name Eusolex HMS by Rona/EM Industries,
Ethylhexyl salicylate sold under the name Neo Heliopan OS by Symrise,

Cinnamic derivatives:

Ethylhexyl methoxycinnamate sold especially under the trade name Parsol MCX by DSM Nutritional Products,

Isopropyl methoxycinnamate,
Isoamyl methoxycinnamate sold under the trade name Neo Heliopan E 1000 by Symrise, Cinoxate,
Diisopropyl methylcinnamate,

β,β-Diphenylacrylate derivatives:

Octocrylene sold especially under the trade name Uvinul N539 by BASF,
Etocrylene sold in particular under the trade name Uvinul N35 by BASF,

Benzophenone derivatives:

Benzophenone-1 sold under the trade name Uvinul 400 by BASF,
Benzophenone-2 sold under the trade name Uvinul D50 by BASF,
Benzophenone-3 or oxybenzone sold under the trade name Uvinul M40 by BASF,
Benzophenone-6 sold under the trade name Helisorb 11 by Norquay,
Benzophenone-8 sold under the trade name Spectra-Sorb UV-24 by American Cyanamid,
Benzophenone-12, N-hexyl 2-(4-diethylamino-2-hydroxybenzoyl)benzoate sold under the trade name Uvinul A+ or in the form of a mixture with octyl methoxycinnamate under the trade name Uvinul A+B by BASF,

Benzylidenecamphor derivatives:

3-Benzylidene Camphor manufactured under the name Mexoryl SD by Chimex,
4-Methylbenzylidene Camphor sold under the name Eusolex 6300 by Merck,
Polyacrylamidomethylbenzylidenecamphor manufactured under the name Mexoryl SW by Chimex,

Phenylbenzotriazole derivatives:
Drometrizole trisiloxane sold under the name Silatrizole by Rhodia Chimie,

Triazine derivatives:

Bis-(Ethylhexyloxyphenyl) methoxyphenyl triazine sold under the trade name Tinosorb S by BASF,
Ethylhexyl triazone sold in particular under the trade name Uvinul T150 by BASF,
Diethylhexyl Butamido Triazone sold under the trade name Uvasorb HEB by Sigma 3V,
2,4,6-Tris(dineopentyl 4'-aminobenzalmalonate)-s-triazine,
2,4,6-Tris(diisobutyl 4'-aminobenzalmalonate)-s-triazine,
2,4-Bis(dineopentyl 4'-aminobenzalmalonate)-6-(n-butyl 4'-aminobenzoate)-s-triazine,
- the triazine silicones substituted by two aminobenzoate groups as those described in EP0841341 in particular 2,4-bis-(n-butyl 4'-aminobenzalmalonate)-6-[(3-{1,3,3,3-tetramethyl-1-[(trimethylsilyloxy]disiloxanyl}propyl)amino]-s-triazine,

Anthranilic derivatives:
Menthyl Anthranilate sold under the trade name Neo Heliopan MA by Symrise,

Imidazoline derivatives:
Ethylhexyl dimethoxybenzylidene dioxoimidazoline propionate,

Benzalmalonate derivatives:

Dineopentyl 4'-methoxybenzalmalonate,
Polyorganosiloxane containing benzalmalonate functions, for instance Polysilicone-15, sold under the trade name Parsol SLX by DSM,

4,4-Diarylbutadiene derivatives:
1,1-dicarboxy(2,2'-dimethylpropyl)-4,4-diphenylbutadiene,

Lipophilic merocyanin derivatives:

- Octyl 5-N,N-diethylamino-2-phenylsulfonyl-2,4-pentadienoate and mixtures thereof.

Preferable lipophilic UV filter may be chosen from:

Butylmethoxydibenzoylmethane,
Ethylhexyl methoxycinnamate,
Ethylhexyl salicylate,
Homosalate,
Butylmethoxydibenzoylmethane,
Octocrylene,
Benzophenone-3,
n-Hexyl 2-(4-diethylamino-2-hydroxybenzoyl)benzoate,
4-Methylbenzylidenecamphor,
Bis(ethylhexyloxyphenyl)methoxyphenyltriazine,
Ethylhexyl triazone,
Diethylhexyl Butamido Triazone,
2,4-bis-(n-butyl 4'-aminobenzalmalonate)-6-[(3-{1,3,3,3-tetramethyl-1-[(trimethylsilyloxy]-disiloxanyl}propyl)amino]-s-triazine,
Drometrizole Trisiloxane,
Polysilicone-15,
1,1-dicarboxy(2,2'-dimethylpropyl)-4,4-diphenylbutadiene, and
mixtures thereof.

More preferable lipophilic UV filters may be chosen from:

Butylmethoxydibenzoylmethane,
Ethylhexyl methoxycinnamate,
Octocrylene,
Ethylhexyl salicylate,
n-Hexyl 2-(4-diethylamino-2-hydroxybenzoyl)benzoate,
Bis(ethylhexyloxyphenyl)methoxyphenyltriazine,
2,4-bis-(n-butyl 4'-aminobenzalmalonate)-6-[(3-{1,3,3,3-tetramethyl-1-[(trimethylsilyloxy]-disiloxanyl}propyl)amino]-s-triazine,
Ethylhexyl triazone,
Diethylhexyl Butamido Triazone,
Drometrizole trisiloxane, and
mixtures thereof.

The cosmetic composition according to the present invention may comprise the organic UV filter(s) in an amount of from 3% to 20% by weight, preferably from 4% to 15% by weight, and more preferably from 5% to 10% by weight, relative to the total weight of the cosmetic composition.

(Fatty Acid)

The fatty phase includes at least one fatty acid. If two or more fatty acids are used, they may be the same or different.

A fatty acid here means an aliphatic monocarboxylic acid, preferably an aliphatic monocarboxylic acid having a long carbon chain. It is preferable that the fatty acid have at least 6 carbon atoms, preferably 7 carbon atoms, and more preferably 8 carbon atoms. The fatty acid may preferably comprise up to 30 carbon atoms, and more preferably up to 20 carbon atoms.

The fatty acid may be selected from saturated or unsaturated, linear or branched fatty acids. As the unsaturated, linear or branched fatty acids, mono-unsaturated, linear or branched fatty acids or polyunsaturated, linear or branched fatty acids may be used. As the unsaturated moiety of the unsaturated, linear or branched fatty acids, a carbon-carbon double bond or a carbon-carbon triple bond may be mentioned.

As the fatty acid, for example, a $C_{8-30}$ saturated, linear or branched fatty acid may be used. As $C_{8-30}$ saturated, linear or branched fatty acids other than lauric acid, mention may be made of caprylic acid ($C_8$), pelargonic acid ($C_9$), capric acid ($C_{10}$), lauric acid ($C_{12}$), myristic acid ($C_{14}$), pentadecanoic acid ($C_{15}$), palmitic acid ($C_{16}$), heptadecanoic acid ($C_{17}$), stearic acid ($C_{18}$), nonadecanoic acid ($C_{19}$), arachidic acid ($C_{20}$), behenic acid ($C_{22}$), and lignoceric acid ($C_{24}$).

On the other hand, as the fatty acid, for example, a $C_{8-30}$ unsaturated, linear or branched fatty acid may also be used. As the $C_{8-30}$ unsaturated, linear or branched fatty acids, mention may be made of palmitoleic acid ($C_{16}$), oleic

acid ($C_{18}$), linoleic acid ($C_{18}$), linolenic acid ($C_{18}$), arachidonic acid ($C_{20}$), and nervonic acid ($C_{24}$).

It is preferable that the fatty acid be a linear or branched, in particular saturated, fatty acid having a carbon chain length of $C_{10-30}$.

It is more preferable that the fatty acid be selected from $C_{13-18}$ fatty acids, preferably $C_{13-18}$ saturated, linear or branched fatty acids. Thus, more preferable fatty acids are myristic acid, pentadecanoic acid, palmitic acid, heptadecanoic acid, and stearic acid. Myristic acid, palmitic acid, and stearic acid are even more preferable.

The fatty acid may be in the form of a free acid or in the form of a salt thereof. As a salt of the fatty acid, mention may be made of an inorganic salt such as an alkali metal salt (a sodium salt, a potassium salt, or the like) and an alkaline earth metal salt (a magnesium salt, a calcium salt, or the like); and an organic salt such as an ammonium salt (a quaternary ammonium salt or the like) and an amine salt (a triethanolamine salt, a triethylamine salt, or the like). A single type of fatty acid salt or a combination of different types of fatty acid salts may be used. Further, a combination of one or more fatty acids in the form of a free acid and one or more fatty acids in the form of a salt may be used, in which one or more types of salts may also be used. It is preferable that at least a part (preferably at least 80%, and more preferably 90%), in particular all, of the fatty acids be in the form of a free acid.

The cosmetic composition according to the present invention may comprise the organic UV filter(s) in an amount of from 0.1% to 5% by weight, preferably from 0.4% to 4% by weight, and more preferably from 0.7% to 3% by weight, relative to the total weight of the cosmetic composition.

{Associative Polymer}

The cosmetic composition according to the present invention includes at least one anionic associative polymer. If two or more associative polymers are used, they may be the same or different. Associative polymers are water-soluble polymers that are capable, in an aqueous medium, of reversibly combining with each other or with other molecules.

Their chemical structure comprises one or more hydrophilic regions and one or more hydrophobic regions characterized by the presence of at least one $C_8$-$C_{30}$ fatty chain.

Among the associative polymers of anionic type that may be mentioned are:

- (I) those comprising at least one hydrophilic unit and at least one fatty-chain allyl ether unit, more particularly those whose hydrophilic unit consists of an ethylenic unsaturated anionic monomer, more particularly of a vinylcarboxylic acid, and most particularly of an acrylic acid or a methacrylic acid, or mixtures thereof, the fatty-chain allyl ether unit of which corresponds to the monomer of formula (XXI) below:

$$CH_2 = CR'CH_2OB_nR \qquad (XXI)$$

in which R' denotes H or $CH_3$, B denotes an ethylenoxy radical, n is zero or denotes an integer ranging from 1 to 100, and R denotes a hydrocarbon-based radical chosen from alkyl, arylalkyl, aryl, alkylaryl, and cycloalkyl radicals, comprising from 8 to 30 carbon atoms, preferably 10 to 24, and even more particularly from 12 to 18 carbon atoms. The unit of formula (XXI) that is more particularly preferred is a unit in which R' denotes H, n is equal to 10, and R denotes a stearyl radical ($C_{18}$).

Anionic associative polymers of this type are described and prepared, according to an emulsion polymerization process, in patent EP-0 216 479.

Among these anionic associative polymers that are particularly preferred according to the invention are polymers formed from 20% to 60% by weight of acrylic acid and/or of methacrylic acid, from 5% to 60% by weight of lower alkyl (meth)acrylates, from 2% to 50% by weight of the fatty-chain allyl ether of formula (XXI), and from 0 to 1% by weight of a crosslinking agent which is a well-known copolymerizable unsaturated polyethylenic monomer, for instance diallyl phthalate, allyl (meth)acrylate, divinylbenzene, (poly)ethylene glycol dimethacrylate, or methylenebisacrylamide.

Among the latter polymers, those most particularly preferred are crosslinked terpolymers of methacrylic acid, of ethyl acrylate and of polyethylene glycol (10 EO) stearyl alcohol ether (Steareth-10), in particular those sold by the company Allied Colloids under the names Salcare SC 80® and Salcare SC 90®, which are aqueous 30% emulsions of a crosslinked terpolymer of methacrylic acid, of ethyl acrylate, and of steareth-10 allyl ether (40/50/10).

- (II) polymers comprising at least one hydrophilic unit of unsaturated olefinic carboxylic acid type, and at least one hydrophobic unit of a type such as a ($C_{10}$-$C_{30}$) alkyl ester of an unsaturated carboxylic acid.

These polymers are preferably chosen from those in which the hydrophilic unit of the unsaturated olefinic carboxylic acid type corresponds to the monomer of formula (XXII) below:

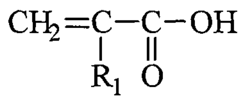

(XXII)

in which $R_1$ denotes H, or $CH_3$ or $C_2H_5$, i.e., acrylic acid, methacrylic acid, or ethacrylic acid units, and whose hydrophobic unit of a type such as a ($C_{10}$-$C_{30}$) alkyl ester of an unsaturated carboxylic acid corresponds to the monomer of formula (XXIII) below:

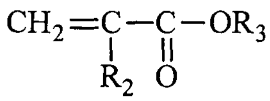

(XXIII)

in which $R_2$ denotes H, or $CH_3$ or $C_2H_5$ (i.e., acrylate, methacrylate, or ethacrylate units) and preferably H (acrylate units) or $CH_3$ (methacrylate units), and $R_3$ denotes a $C_{10}$-$C_{30}$ and preferably $C_{12}$-$C_{22}$ alkyl radical.

($C_{10}$-$C_{30}$)alkyl esters of unsaturated carboxylic acids in accordance with the invention comprise, for example, lauryl acrylate, stearyl acrylate, decyl acrylate, isodecyl acrylate, and dodecyl acrylate, and the corresponding methacrylates, lauryl methacrylate, stearyl methacrylate, decyl methacrylate, isodecyl methacrylate, and dodecyl methacrylate.

Anionic polymers of this type are described and prepared, for example, according to patents US 3 915 921 and US 4 509 949.

According to a preferred embodiment, these polymers are crosslinked.

Among the anionic associative polymers of this type that will be used more particularly are polymers formed from a monomer mixture comprising:

(i) essentially acrylic acid,
(ii) an ester of formula (XXIII) described above and in which $R_2$ denotes H or $CH_3$, and $R_3$ denotes an alkyl radical containing from 12 to 22 carbon atoms, and
(iii) a crosslinking agent, which is a well-known copolymerizable unsaturated polyethylenic monomer, for instance diallyl phthalate, allyl (meth)acrylate, divinylbenzene, (poly)ethylene glycol dimethacrylate, and methylenebisacrylamide.

Among the anionic associative polymers of this type, use will be made more particularly of those formed from 95% to 60% by weight of acrylic acid (hydrophilic unit), 4% to 40% by weight of $C_{10}$-$C_{30}$ alkyl acrylate (hydrophobic unit), and 0 to 6% by weight of crosslinking polymerizable monomer, or alternatively those formed from 98% to 96% by weight of acrylic acid (hydrophilic unit), 1% to 4% by weight of $C_{10}$-$C_{30}$ alkyl acrylate (hydrophobic unit), and 0.1% to 0.6% by weight of crosslinking polymerizable monomer such as those described previously.

Among the said above polymers, those most particularly preferred according to the present invention are acrylate/$C_{10-30}$ alkyl acrylate copolymers (INCI name: Acrylates/C10-30 Alkyl Acrylate Crosspolymer), such as the products sold by the company Lubrizol under the trade names Pemulen TR1®, Pemulen TR2®, Carbopol 1382®, and Carbopol EDT2020®, and even more preferentially Pemulen TR2®,.

- (III) maleic anhydride/$C_{30}$-$C_{38}$ $\alpha$-olefin/alkyl maleate terpolymers, such as the product (maleic anhydride/$C_{30}$-$C_{38}$ $\alpha$-olefin/isopropyl maleate) sold under the name Performa V 1608® by the company New Phase Technologies.

- (IV) acrylic terpolymers comprising:

(a) about 20% to 70% by weight of a carboxylic acid containing $\alpha,\beta$-monoethylenic unsaturation,
(b) about 20% to 80% by weight of a non-surfactant monomer containing $\alpha,\beta$-monoethylenic unsaturation other than (a), and
(c) about 0.5% to 60% by weight of a nonionic monourethane which is the product of reaction of a monohydric surfactant with a monoisocyanate containing monoethylenic unsaturation,

such as those described in patent application EP-A-0 173 109 and more particularly the terpolymer described in Example 3, namely a methacrylic acid/methyl acrylate/behenyl alcohol dimethyl-meta-isopropenylbenzylisocyanate ethoxylated (40 EO) terpolymer, as an aqueous 25% dispersion.

- (V) copolymers comprising among their monomers a carboxylic acid containing α,β-monoethylenic unsaturation and an ester of a carboxylic acid containing α,β-monoethylenic unsaturation and of an oxyalkylenated fatty alcohol.

Preferentially, these compounds also comprise as a monomer an ester of an α,β-monoethylenically unsaturated carboxylic acid and of a $C_1$-$C_4$ alcohol.
An example of a compound of this type that may be mentioned is Aculyn 22® sold by the company Röhm & Haas, which is a methacrylic acid/ethyl acrylate/oxyalkylenated stearyl methacrylate terpolymer.
Among the associative polymers of cationic type that may be mentioned are:

- (I) cationic associative polyurethanes, the family of which has been described by the Applicant in French patent application No. 00/09609; these may be represented by the general formula (XXIV) below:

R-X-(P)n-[L-(Y)m]r-L'-(P')p-X'-R' (XXIV)

in which:

R and R', which may be identical or different, represent a hydrophobic group or a hydrogen atom;
X and X', which may be identical or different, represent a group comprising an amine function optionally bearing a hydrophobic group, or alternatively a group L";
L, L', and L", which may be identical or different, represent a group derived from a diisocyanate;
P and P', which may be identical or different, represent a group comprising an amine function optionally bearing a hydrophobic group;
Y represents a hydrophilic group;
r is an integer between 1 and 100, preferably between 1 and 50, and in particular between 1 and 25;
n, m, and p each range, independently of each other, from 0 to 1000; and
the molecule contains at least one protonated or quaternized amine function and at least one hydrophobic group.

In one preferred embodiment of these polyurethanes, the only hydrophobic groups are the groups R and R' at the chain ends.
One preferred family of cationic associative polyurethanes is the one corresponding to formula (XXIV) described above and in which:

R and R' both independently represent a hydrophobic group,
X and X' each represent a group L",
n and p are between 1 and 1000, and
L, L', L", P, P', Y, and m have the meanings given above.

Another preferred family of cationic associative polyurethanes is the one corresponding to formula (XXIV) above in which:
R and R' both independently represent a hydrophobic group, X and X' each represent a group L", n and p are 0, and L, L', L", Y, and m have the meanings given above.
The fact that n and p are 0 means that these polymers do not comprise units derived from a monomer containing an amine function, incorporated into the polymer during the polycondensation. The protonated amine functions of these polyurethanes result from the hydrolysis of excess isocyanate functions, at the chain end, followed by alkylation of the primary amine functions formed with alkylating agents containing a hydrophobic group, i.e., compounds of the type RQ or R'Q, in which R and R' are as defined above and Q denotes a leaving group such as a halide, a sulfate, etc.
Yet another preferred family of cationic associative polyurethanes is the one corresponding to formula (XXIV) above in which:

R and R' both independently represent a hydrophobic group,
X and X' both independently represent a group comprising a quaternary amine,
n and p are zero, and
L, L', Y, and m have the meanings given above.

The number-average molecular mass of the cationic associative polyurethanes is preferably between 400 and 500 000, in particular between 1000 and 400 000, and ideally between 1000 and 300 000.
The expression "hydrophobic group" means a radical or polymer containing a saturated or unsaturated, linear

or branched hydrocarbon-based chain, which may contain one or more heteroatoms such as P, O, N, or S, or a radical containing a perfluoro or silicone chain. When the hydrophobic group denotes a hydrocarbon-based radical, it comprises at least 10 carbon atoms, preferably from 10 to 30 carbon atoms, in particular from 12 to 30 carbon atoms, and more preferably from 18 to 30 carbon atoms.

Preferentially, the hydrocarbon-based group is derived from a monofunctional compound.

By way of example, the hydrophobic group may be derived from a fatty alcohol such as stearyl alcohol, dodecyl alcohol, or decyl alcohol. It may also denote a hydrocarbon-based polymer, for instance polybutadiene.

When X and/or X' denote(s) a group comprising a tertiary or quaternary amine, X and/or X' may represent one of the following formulae:

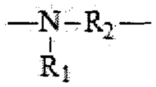 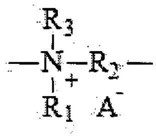 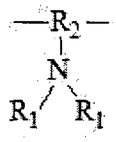 or 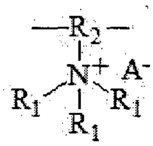 for X

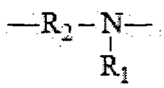 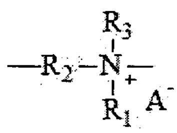 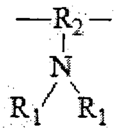 or 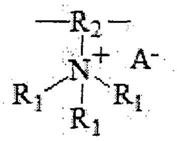 for X'

in which:

- $R_2$ represents a linear or branched alkylene radical containing from 1 to 20 carbon atoms, optionally comprising a saturated or unsaturated ring, or an arylene radical, one or more of the carbon atoms possibly being replaced with a heteroatom chosen from N, S, O, and P;
- $R_1$ and $R_3$, which may be identical or different, denote a $C_1$-$C_{30}$ alkyl or alkenyl radical or an aryl radical, at least one of the carbon atoms possibly being replaced with a heteroatom chosen from N, S, O, and P; and
- $A^-$ is a physiologically acceptable counterion.

The groups L, L', and L" represent a group of formula:

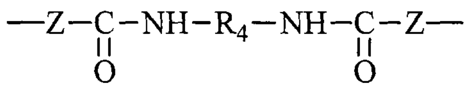

in which:

- Z represents -O-, -S-, or -NH-; and
- $R_4$ represents a linear or branched alkylene radical containing from 1 to 20 carbon atoms, optionally comprising a saturated or unsaturated ring, or an arylene radical, one or more of the carbon atoms possibly being replaced with a heteroatom chosen from N, S, O, and P.

The groups P and P' comprising an amine function may represent at least one of the following formulae:

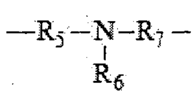 or 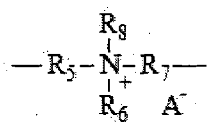

or

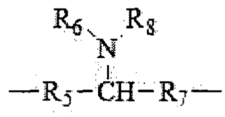

or

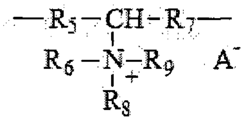

or

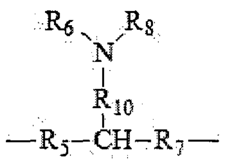

or

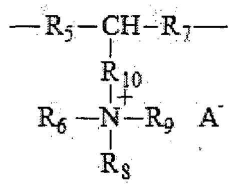

in which:

$R_5$ and $R_7$ have the same meaning as $R_2$ defined above;
$R_6$, $R_8$, and $R_9$ have the same meanings as $R_1$ and $R_3$ defined above;
$R_{10}$ represents a linear or branched, optionally unsaturated alkylene group possibly containing one or more heteroatoms chosen from N, O, S, and P; and
$A^-$ is a physiologically acceptable counterion.

As regards the meaning of Y, the term "hydrophilic group" means a polymeric or non-polymeric water-soluble group.
By way of example, when it is not a polymer, mention may be made of ethylene glycol, diethylene glycol, and propylene glycol.
When it is a hydrophilic polymer, in accordance with one preferred embodiment, mention may be made, for example, of polyethers, sulfonated polyesters, sulfonated polyamides, or a mixture of these polymers. The hydrophilic compound is preferentially a polyether and especially a poly(ethylene oxide) or poly(propylene oxide).
The cationic associative polyurethanes of formula (XXIV) that may be used according to the invention are formed from diisocyanates and from various compounds with functions containing a labile hydrogen. The functions containing a labile hydrogen may be alcohol, primary or secondary amine, or thiol functions, giving, after reaction with the diisocyanate functions, polyurethanes, polyureas, and polythioureas, respectively. The expression "polyurethanes" that may be used according to the present invention encompasses these three types of polymer, namely polyurethanes per se, polyureas, and polythioureas, and also copolymers thereof.
A first type of compound involved in the preparation of the polyurethane of formula (XXIV) is a compound comprising at least one unit containing an amine function. This compound may be multifunctional, but the compound is preferentially difunctional, that is to say that, according to one preferential embodiment, this compound comprises two labile hydrogen atoms borne, for example, by a hydroxyl, primary amine, secondary amine, or thiol function. A mixture of multifunctional and difunctional compounds in which the percentage of multifunctional compounds is low may also be used.
As mentioned above, this compound may comprise more than one unit containing an amine function. In this case, it is a polymer bearing a repetition of the unit containing an amine function.
Compounds of this type may be represented by one of the following formulae:

HZ-(P)n-ZH

or

HZ-(P')p-ZH

in which Z, P, P', n, and p are as defined above.
Examples of compounds containing an amine function that may be mentioned include N-methyldiethanolamine,

N-tert-butyldiethanolamine, and N-sulfoethyldiethanolamine.

The second compound included in the preparation of the polyurethane of formula (XVIII) is a diisocyanate corresponding to the formula:

$$O=C=N-R_4-N=C=O$$

in which $R_4$ is as defined above.

By way of example, mention may be made of methylenediphenyl diisocyanate, methylenecyclohexane diisocyanate, isophorone diisocyanate, tolylene diisocyanate, naphthalene diisocyanate, butane diisocyanate, and hexane diisocyanate.

A third compound involved in the preparation of the polyurethane of formula (XXIV) is a hydrophobic compound intended to form the terminal hydrophobic groups of the polymer of formula (XXIV).

This compound consists of a hydrophobic group and of a function containing a labile hydrogen, for example, a hydroxyl, primary or secondary amine, or thiol function

By way of example, this compound may be a fatty alcohol such as, in particular, stearyl alcohol, dodecyl alcohol, or decyl alcohol. When this compound comprises a polymeric chain, it may be, for example, $\alpha$-hydroxylated hydrogenated polybutadiene.

The hydrophobic group of the polyurethane of formula (XXIV) may also result from the quaternization reaction of the tertiary amine of the compound comprising at least one tertiary amine unit. Thus, the hydrophobic group is introduced via the quaternizing agent. This quaternizing agent is a compound of the type RQ or R'Q, in which R and R' are as defined above and Q denotes a leaving group such as a halide, a sulfate, etc.

The cationic associative polyurethane may also comprise a hydrophilic block. This block is provided by a fourth type of compound involved in the preparation of the polymer. This compound may be multifunctional. It is preferably difunctional. It is also possible to have a mixture in which the percentage of multifunctional compound is low.

The functions containing a labile hydrogen are alcohol, primary or secondary amine, or thiol functions. This compound may be a polymer terminated at the chain ends with one of these functions containing a labile hydrogen.

By way of example, when it is not a polymer, mention may be made of ethylene glycol, diethylene glycol, and propylene glycol.

When it is a hydrophilic polymer, mention may be made, for example, of polyethers, sulfonated polyesters, and sulfonated polyamides, or a mixture of these polymers. The hydrophilic compound is preferentially a polyether and especially a poly(ethylene oxide) or poly(propylene oxide).

The hydrophilic group termed Y in formula (XXIV) is optional. Specifically, the units containing a quaternary amine or protonated function may suffice to provide the solubility or water-dispersibility required for this type of polymer in an aqueous solution.

Although the presence of a hydrophilic group Y is optional, cationic associative polyurethanes comprising such a group are, however, preferred.

- (II) quaternized cellulose derivatives and polyacrylates containing non-cyclic amine side groups.

The quaternized cellulose derivatives are, in particular:

- quaternized celluloses modified with groups comprising at least one fatty chain, such as alkyl, arylalkyl, or alkylaryl groups comprising at least 8 carbon atoms, or mixtures thereof,
- quaternized hydroxyethylcelluloses modified with groups comprising at least one fatty chain, such as alkyl, arylalkyl, or alkylaryl groups comprising at least 8 carbon atoms, or mixtures thereof.

The alkyl radicals borne by the above quaternized celluloses or hydroxyethylcelluloses preferably contain from 8 to 30 carbon atoms. The aryl radicals preferably denote phenyl, benzyl, naphthyl, or anthryl groups.

Examples of quaternized alkylhydroxyethylcelluloses containing $C_8$-$C_{30}$ fatty chains that may be mentioned include the products Quatrisoft LM 200®, Quatrisoft LM-X 529-18-A®, Quatrisoft LM-X 529-18B® ($C_{12}$ alkyl), and Quatrisoft LM-X 529-8® ($C_{18}$ alkyl) sold by the company Amerchol, and the products Crodacel QM®, Crodacel QL® ($C_{12}$ alkyl), and Crodacel QS® ($C_{18}$ alkyl) sold by the company Croda.

The amphoteric associative polymers are preferably chosen from those comprising at least one non-cyclic cationic unit. Even more particularly, the ones that are preferred are those prepared from or comprising 1 to 20 mol%, preferably 1.5 to 15 mol%, and even more particularly 1.5 to 6 mol% of fatty-chain monomer relative to the total number of moles of monomers.

The amphoteric associative polymers that are preferred according to the invention comprise or are prepared by copolymerizing:

1) at least one monomer of formula (XXV) or (XXVI):

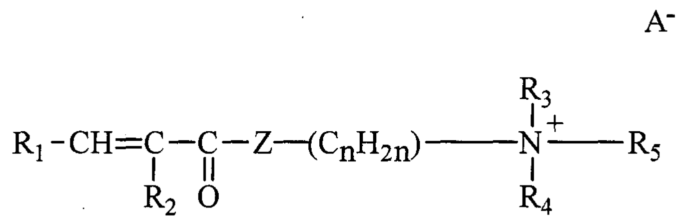

(XXV)

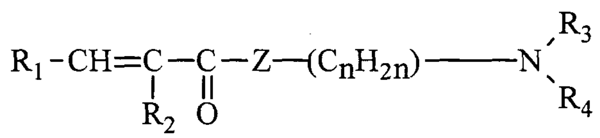

(XXVI)

in which $R_1$ and $R_2$, which may be identical or different, represent a hydrogen atom or a methyl radical, $R_3$, $R_4$, and $R_5$, which may be identical or different, represent a linear or branched alkyl radical containing from 1 to 30 carbon atoms,
Z represents an NH group or an oxygen atom,
n is an integer from 2 to 5,
$A^-$ is an anion derived from an organic or mineral acid, such as a methosulfate anion or a halide such as chloride or bromide;

2) at least one monomer of formula (XXVII):

$R_6$-CH=C$R_7$-COOH (XXVII)

in which $R_6$ and $R_7$, which may be identical or different, represent a hydrogen atom or a methyl radical; and

3) at least one monomer of formula (XXVIII):

$R_6$-CH=C$R_7$-COX$R_8$ (XXVIII)

in which $R_6$ and $R_7$, which may be identical or different, represent a hydrogen atom or a methyl radical, X denotes an oxygen or nitrogen atom, and $R_8$ denotes a linear or branched alkyl radical containing from 1 to 30 carbon atoms;
at least one of the monomers of formula (XXV), (XXVI), or (XXVIII) comprising at least one fatty chain.

The monomers of formulae (XXV) and (XXVI) of the present invention are preferably chosen from the group consisting of:

- dimethylaminoethyl methacrylate, dimethylaminoethyl acrylate,
- diethylaminoethyl methacrylate, diethylaminoethyl acrylate,
- dimethylaminopropyl methacrylate, dimethylaminopropyl acrylate, and
- dimethylaminopropylmethacrylamide, dimethylaminopropylacrylamide,

these monomers optionally being quaternized, for example, with a $C_1$-$C_4$ alkyl halide or a $C_1$-$C_4$ dialkyl sulfate.

More particularly, the monomer of formula (XXV) is chosen from acrylamido-propyltrimethylammonium chloride and methacrylamidopropyltrimethylammonium chloride.

The monomers of formula (XXVII) of the present invention are preferably chosen from the group consisting of acrylic acid, methacrylic acid, crotonic acid, and 2-methylcrotonic acid. More particularly, the monomer of formula (XXI) is acrylic acid.

The monomers of formula (XXVIII) of the present invention are preferably chosen from the group formed from $C_{12}$-$C_{22}$ and more particularly $C_{16}$-$C_{18}$ alkyl acrylates or methacrylates.

The monomers constituting the fatty-chain amphoteric polymers of the invention are preferably already neutralized and/or quaternized.

The ratio of the number of cationic charges/anionic charges is preferably equal to about 1.

The amphoteric associative polymers according to the invention preferably comprise from 1 mol% to 10 mol% of the monomer comprising a fatty chain (monomer of formula (XXV), (XXVI), or (XXVIII)), and preferably from 1.5 mol% to 6 mol%.

The weight-average molecular weights of the amphoteric associative polymers according to the invention may range from 500 to 50 000 000 and are preferably between 10 000 and 5 000 000.

The amphoteric associative polymers according to the invention may also contain other monomers such as nonionic monomers and in particular such as $C_1$-$C_4$ alkyl acrylates or methacrylates.

Amphoteric associative polymers according to the invention are described and prepared, for example, in patent application WO 98/44012.

Among the amphoteric associative polymers according to the invention, the ones that are preferred are acrylic acid/(meth)acrylamidopropyltrimethylammonium chloride/stearyl methacrylate terpolymers.

The associative polymers of nonionic type that may be used according to the invention are preferably chosen from:

- (1) celluloses modified with groups comprising at least one fatty chain;

examples that may be mentioned include:

- hydroxyethylcelluloses modified with groups comprising at least one fatty chain, such as alkyl, arylalkyl, or alkylaryl groups, or mixtures thereof, and in which the alkyl groups are preferably $C_8$-$C_{22}$, for instance the product Natrosol Plus Grade 330 CS® ($C_{16}$ alkyl) sold by the company Aqualon, or the product Bermocoll EHM 100® sold by the company Berol Nobel,
- hydroxyethylcelluloses modified with alkylphenyl polyalkylene glycol ether groups, such as the product Amercell Polymer HM-1500® (nonylphenyl polyethylene glycol (15) ether) sold by the company Amerchol.
- (2) hydroxypropyl guars modified with groups comprising at least one fatty chain, such as the product Esaflor HM 22® ($C_{22}$ alkyl chain) sold by the company Lamberti, and the products RE210-18® ($C_{14}$ alkyl chain) and RE205-1® ($C_{20}$ alkyl chain) sold by the company Rhône-Poulenc.
- (3) copolymers of vinylpyrrolidone and of fatty-chain hydrophobic monomers; examples that may be mentioned include:
- the products Antaron V216® or Ganex V216® (vinylpyrrolidone/hexadecene copolymer) sold by the company ISP.
- the products Antaron V220® or Ganex V220® (vinylpyrrolidone/eicosene copolymer) sold by the company ISP.
- (4) copolymers of $C_1$-$C_6$ alkyl methacrylates or acrylates and of amphiphilic monomers comprising at least one fatty chain, for instance the oxyethylenated methyl acrylate/stearyl acrylate copolymer sold by the company Goldschmidt under the name Antil 208®.
- (5) copolymers of hydrophilic methacrylates or acrylates and of hydrophobic monomers comprising at least one fatty chain, for instance the polyethylene glycol methacrylate/lauryl methacrylate copolymer.
- (6) polyurethane polyethers comprising in their chain both hydrophilic blocks usually of polyoxyethylenated nature and hydrophobic blocks, which may be aliphatic sequences alone and/or cycloaliphatic and/or aromatic sequences.
- (7) polymers with an aminoplast ether backbone containing at least one fatty chain, such as the Pure Thix® compounds sold by the company Sud-Chemie.

Preferably, the polyurethane polyethers comprise at least two hydrocarbon-based lipophilic chains containing from 6 to 30 carbon atoms, separated by a hydrophilic block, the hydrocarbon-based chains possibly being pendent chains or chains at the end of the hydrophilic block. In particular, it is possible for one or more pendent chains to be included. In addition, the polymer may comprise a hydrocarbon-based chain at one end or at both ends of a hydrophilic block.

The polyurethane polyethers may be multiblock, in particular in triblock form. The hydrophobic blocks may be at each end of the chain (for example: triblock copolymer containing a hydrophilic central block) or distributed both at the ends and in the chain (for example, multiblock copolymer). These same polymers may also be graft polymers or star polymers.

The nonionic fatty-chain polyurethane polyethers may be triblock copolymers in which the hydrophilic block is a polyoxyethylenated chain comprising from 50 to 1000 oxyethylene groups. The nonionic polyurethane polyethers comprise a urethane linkage between the hydrophilic blocks, whence arises the name.

By extension, also included among the nonionic fatty-chain polyurethane polyethers are those in which the hydrophilic blocks are linked to the lipophilic blocks via other chemical bonds.

As examples of nonionic fatty-chain polyurethane polyethers that may be used in the invention, it is also possible to use Rheolate 205® containing a urea function, sold by the company Rheox, or Rheolate® 208, 204, or 212, and also

Acrysol RM 184®.

Mention may also be made of the product Elfacos T210® containing a $C_{12-14}$ alkyl chain, and the product Elfacos T212® containing a $C_{18}$ alkyl chain, from Akzo.

The product DW 1206B® from Rohm & Haas containing a $C_{20}$ alkyl chain and a urethane bond, sold at a solids content of 20% in water, may also be used.

It is also possible to use solutions or dispersions of these polymers, especially in water or in aqueous-alcoholic medium. Examples of such polymers that may be mentioned are Rheolate® 255, Rheolate® 278, and Rheolate® 244 sold by the company Rheox. The products DW 1206F and DW 1206J sold by the company Röhm & Haas may also be used.

The polyurethane polyethers that may be used according to the invention are in particular those described in the article by G. Fonnum, J. Bakke, and Fk. Hansen - Colloid Polym. Sci 271, 380.389 (1993).

It is even more particularly preferred to use a polyurethane polyether that may be obtained by polycondensation of at least three compounds comprising (i) at least one polyethylene glycol comprising from 150 to 180 mol of ethylene oxide, (ii) stearyl alcohol or decyl alcohol, and (iii) at least one diisocyanate.

Such polyurethane polyethers are sold especially by the company Röhm & Haas under the names Aculyn 46® and Aculyn 44® [Aculyn 46® is a polycondensate of polyethylene glycol containing 150 or 180 mol of ethylene oxide, of stearyl alcohol, and of methylenebis(4-cyclohexyl isocyanate) (SMDI), at 15% by weight in a matrix of maltodextrin (4%) and water (81%); Aculyn 44® is a polycondensate of polyethylene glycol containing 150 or 180 mol of ethylene oxide, of decyl alcohol, and of methylenebis(4-cyclohexyl isocyanate) (SMDI), at 35% by weight in a mixture of propylene glycol (39%) and water (26%)].

Among the said above associative polymers, those most particularly preferred according to the present invention are acrylate/$C_{10-30}$ alkyl acrylate copolymers (INCI name: Acrylates/C10-30 Alkyl Acrylate Crosspolymer), such as the products sold by the company Lubrizol under the trade names Pemulen TR1®, Pemulen TR2®, Carbopol 1382®, and Carbopol EDT2020®, and even more preferentially Pemulen TR2®.

The cosmetic composition according to the present invention may comprise the associative polymer(s) in an amount of from 0.01% to 5% by weight, preferably from 0.5% to 3% by weight, and more preferably from 0.1% to 1% by weight, relative to the total weight of the cosmetic composition.

{Hollow Particle}

The cosmetic composition according to the present invention includes at least one hollow particle. If two or more hollow particles are used, they may be the same or different.

The hollow particles according to the invention may have a particle size which ranges generally from 100 to 380 nm, preferably from 150 to 375 nm, more preferably from 190 to 350 nm, and more particularly from 251 to 325 nm, the particle size being measured by a Brookhaven BI-90 photon correlation spectrometer.

The hollow particle according to the present invention includes at least one hollow.

For a given particle size, the hollow particles according to the invention, in general, possess a maximum hollow fraction. The hollow particles preferably contain a void fraction of 0.1% to 50% and more preferably of 5% to 50%. The void fractions are determined by comparing the volume occupied by the hollow particles after having been compacted from a diluted dispersion in a centrifuge, relative to the volume of non-void particles in the same composition.

Hollow particles according to the invention may be obtained from particles comprising at least one polymer for the core and at least one polymer for the shell. The core polymer and the shell polymer may be obtained from a single polymerization step or from a sequence of polymerization steps.

The hollow particles according to the invention may be prepared by the conventional techniques of emulsion polymerization. Such processes are described especially in patents US 4,427,836, US 4,469,825, US 4,594,363, US 4,677,003, US 4,920,160, and US 4,970,241 or by the conventional techniques of polymerization that are described in the following patents and patent applications: EP267726, EP331421, US 490,229, and US 5,157,084.

The monomers used for the shell of the hollow particles are preferably constituted of one or more unsaturated nonionic ethylenic units. Optionally one or more monoethylenically unsaturated monomers containing at least one carboxylic acid group may be polymerized in the shell.

The monomers constituting the shell may be selected such that they exhibit a glass transition temperature (Tg) which is sufficiently high to withstand the void of the hollow particle. Preferably the glass transition temperature is greater than 50°C, more preferably greater than 60°C, and more preferably still greater than 70°C. This temperature Tg may be determined by DSC (differential scanning calorimetry).

The monomers used in the emulsion polymerization in the core polymer of the hollow particles of the invention are preferably constituted of one or more monoethylenically unsaturated monomers containing at least one carboxylic acid group. Preferably the core comprises at least 5% by weight of monoethylenically unsaturated monomer containing at least one carboxylic acid group, relative to the total weight of the core monomers.

The core polymer may for example be obtained by emulsion homopolymerization of the monoethylenically

unsaturated monomer containing at least one acid group or by copolymerization of two or three monoethylenically unsaturated monomers containing at least one acid group. Preferably the monoethylenically unsaturated monomer containing at least one acid group is copolymerized with one or more ethylenically unsaturated nonionic monomers.

The core polymer or the shell polymer may contain from 0.1% to 20% by weight, preferably from 0.1% to 3% by weight, of polyethylenically unsaturated monomers such as ethylene glycol di(meth)acrylate, allyl (meth)acrylate, 1,3-butanediol di(meth)acrylate, diethylene glycol di(meth)acrylate, trimethylolpropane tri(meth)acrylate, or divinylbenzene, relative to the total weight of core monomers. Alternatively the core polymer or the shell polymer may optionally contain from 0.1% to 60% by weight of butadiene, relative to the total weight of core monomers.

The monoethylenically unsaturated monomers containing at least one carboxylic acid group include, for example:

acrylic acid, methacrylic acid, acryloyloxypropionic acid, (meth)acryloyloxypropionic acid, itaconic acid, aconitic acid, maleic acid or maleic anhydride, fumaric acid, crotonic acid, monomethyl maleate, monomethyl fumarate, and monomethyl itaconate.

Use will be made more particularly of a monomer selected from acrylic acid and methacrylic acid.

The monoethylenically unsaturated nonionic monomers include, for example:

styrene, vinyltoluene, vinyl acetate, vinyl chloride, vinylidene chloride, acrylonitrile, (meth)acrylamide, $C_1$-$C_{20}$ alkyl esters of (meth)acrylic acid, and ($C_3$-$C_{20}$) alkenyl esters of (meth)acrylic acid, such as methyl (meth)acrylate, ethyl (meth)acrylate, butyl (meth)acrylate, 2-ethylhexyl (meth)acrylate, benzyl (meth)acrylate, lauryl (meth)acrylate, oleyl (meth)acrylate, palmityl (meth)acrylate, and stearyl (meth)acrylate. According to the invention, the term (meth)acrylic will denote the general expression encompassing both methacrylic or acrylic. The term (meth)acrylate will denote the general expression encompassing both methacrylate or acrylate.

The void part of the core of the hollow particles is preferably produced by swelling the core with a swelling agent comprising one or more volatile compounds. The agent penetrates the shell in order to swell the core. The volatile components of the swelling agent may be subsequently removed by drying the hollow particles, thus creating a void within the said particles. The agent is preferably an aqueous base. Mention may be made, for example, of ammonia, ammonium hydroxide, alkali metal hydroxides such as sodium hydroxide, and volatile amines such as trimethylamine or triethylamine.

The hollow particles may be introduced into the composition of the invention with the swelling agent. In that case the volatile compounds are removed when the composition is dried. The hollow particles may also be added to the composition after the volatile compounds of the swelling agent have been removed.

The hollow particles which can be used according to the invention are those described in patent US 5,663,213 and patent application EP1092421.

Typically, the hollow particles are provided as part of an aqueous dispersion that is generally stabilized with at least one emulsifier. Thus, the hollow particles may be in the form of a latex.

According to one particular embodiment of the invention, the hollow particles used will be those constituted of a copolymer of styrene and (meth)acrylic acid or one of its $C_1$-$C_{20}$ alkyl esters under the INCI name Styrene/Acrylates Copolymer, such as the product sold under the trade name Sunspheres Powder by the company Rohm & Haas, which is an aqueous dispersion containing 86% of Styrene/Acrylates Copolymer in a mixture of 11% of PEG-8 Laurate, 2.5% of water, and 0.5% of Sodium Dodecylbenzenesulfonate.

The cosmetic composition according to the present invention may comprise the hollow particle(s) in an amount of from 0.1% to 5% by weight, preferably from 0.5% to 4% by weight, and more preferably from 1% to 3% by weight, relative to the total weight of the cosmetic composition.

(Galenical Forms)

The compositions according to the invention can be prepared according to the techniques well known to those skilled in the art for preparing O/W emulsions. They may be in the form of a milk, of a cream or of a cream gel. They may optionally be packaged as an aerosol and may be in the form of a spray.

The emulsification processes that may be used are of paddle or impeller, rotor-stator and high-pressure homogenizer (HPH) type.

To obtain stable emulsions with a low content of emulsifying compounds (oil/emulsifier ratio > 25), it is possible to make the dispersion in concentrated phase and then to dilute the dispersion with the rest of the aqueous phase.

It is also possible, via HPH (between 50 and 800 bar), to obtain stable dispersions with drop sizes which can fall to 100 nm.

The emulsions generally comprise at least one emulsifier chosen from amphoteric, anionic, cationic or nonionic emulsifiers, used alone or as a mixture. The emulsifiers are appropriately chosen according to the emulsion to be obtained O/W). The emulsions may also contain stabilizers of other types, for instance fillers, or gelling or thickening polymers.

For the O/W emulsions, examples of emulsifiers that may be mentioned include non-ionic emulsifiers such as oxyalkylenated (more particularly polyoxyethylenated) esters of fatty acids and of glycerol; oxyalkylenated esters of fatty acids and of sorbitan; oxyalkylenated (oxyethylenated and/or oxypropylenated) esters of fatty acids, such as the PEG-100 stearate/glyceryl stearate mixture sold, for example, by ICI under the name Arlacel 165; oxyalkylenated (oxyethylenated and/or oxypropylenated) ethers of fatty alcohols; esters of sugars, such as sucrose stearate; or ethers of fatty alcohol and of sugar, in particular alkyl polyglucosides (APGs), such as decyl glucoside and lauryl glucoside, sold, for example, by the company Henkel under the respective names Plantaren 2000 and Plantaren 1200, cetostearyl glucoside, optionally as a mixture with cetostearyl alcohol, sold, for example, under the name Montanov 68 by the company SEPPIC, under the name Tegocare CG90 by the company Goldschmidt and under the name Emulgade KE3302 by the company Henkel, and also arachidyl glucoside, for example in the form of the mixture of arachidyl alcohol, behenyl alcohol and arachidyl glucoside, sold under the name Montanov 202 by the company SEPPIC. According to one particular embodiment of the invention, the mixture of the alkylpolyglucoside as defined above with the corresponding fatty alcohol can be in the form of a self-emulsifying composition, for example as described in document WO-A-92/06778.

Among the other emulsion stabilizers, use may also be made of isophthalic acid or sulfoisophthalic acid polymers, and in particular phthalate/sulfoisophthalate/glycol copolymers, for example the diethylene glycol/phthalate/isophthalate/1,4-cyclohexanedimethanol copolymer (INCI name: Polyester-5) sold under the names Eastman AQ Polymer (AQ35S, AQ38S, AQ55S and AQ48 Ultra) by the company Eastman Chemical.

Among the other emulsion stabilizers, mention may also be made of hydrophobically modified 2-acrylamido-2-methylpropanesulfonic acid polymers such as those described in patent application EP 1 069 142.

The aqueous phase of the latter can comprise a non-ionic vesicular dispersion prepared according to known processes (Bangham, Standish and Watkins, J. Mol. Biol., 13, 238 (1965), FR 2 315 991 and FR 2 416 008).

(Optional Ingredients)

The compositions in accordance with the present invention may also comprise one or more standard cosmetic adjuvants chosen from oils, waxes, organic solvents, hydrophilic UV-screening agents, ionic or nonionic, hydrophilic or lipophilic thickeners, softeners, humectants, opacifiers, stabilizers, emollients, silicones, antifoams, fragrances, preserving agents, surfactants, active agents, fillers, colouring agents, polymers, propellants, acidifying or basifying agents, or any other ingredient usually used in cosmetics and/or dermatology.

Needless to say, a person skilled in the art will take care to select the optional adjuvant(s) added to the composition according to the invention such that the advantageous properties intrinsically associated with the composition in accordance with the invention are not, or are not substantially, adversely affected by the envisaged addition.

Among the organic solvents that may be mentioned are lower alcohols and polyols. The latter can be chosen from glycols and glycol ethers, such as ethylene glycol, propylene glycol, butylene glycol, dipropylene glycol or diethylene glycol.

Hydrophilic thickeners that may be mentioned include carboxyvinyl polymers such as the Carbopol products (carbomers) and the Pemulen products (acrylate/C10-C30-alkyl acrylate copolymer); polyacrylamides, for instance the crosslinked copolymers sold under the names Sepigel 305 (CTFAname: polyacrylamide/C13-C14 isoparaffin/Laureth 7) or Simulgel 600 (CTFA name: acrylamide/sodium acryloyldimethyl taurate copolymer/isohexadecane/polysorbate 80) by the company SEPPIC; 2-acrylamido-2-methylpropanesulfonic acid polymers and copolymers, optionally crosslinked and/or neutralized, for instance poly(2-acrylamido-2-methylpropanesulfonic acid) sold by the company Clariant under the trade name Hostacerin AMPS (CTFA name: ammonium polyacryloyldimethyl taurate) or Simulgel 800 sold by the company SEPPIC (CTFA name: sodium polyacryloyldimethyl taurate/polysorbate 80/sorbitan oleate); copolymers of 2-acrylamido-2-methylpropanesulfonic acid and of hydroxyethyl acrylate, for instance Simulgel NS and Sepinov EMT 10 sold by the company SEPPIC; cellulose derivatives such as hydroxyethyl cellulose; polysaccharides and especially gums such as xanthan gum; and mixtures thereof.

Lipophilic thickeners that may be mentioned include synthetic polymers such as poly(C10-C30 alkyl acrylates) sold under the name Intelimer IPA 13-1 and Intelimer IPA 13-6 by the company Landec, or modified clays such as hectorite and its derivatives, for instance the products sold under the name Bentone.

The compositions according to the invention may also furthermore comprise additional cosmetic and dermatological active agents.

Mention may be made, among active agents, of:

- vitamins (A, C, E, K, PP, and the like) and their derivatives or precursors, alone or as mixtures;
- antioxidants;
- free-radical scavengers;
- antiglycation agents;
- soothing agents;

- NO-synthase inhibitors;
- agents which stimulate the synthesis of dermal or epidermal macromolecules and/or which prevent their decomposition;
- agents which stimulate the proliferation of fibroblasts;
- agents which stimulate the proliferation of keratinocytes;
- muscle relaxants;
- tensioning agents;
- mattifying agents;
- keratolytic agents;
- desquamating agents;
- moisturizers, for instance polyols such as glycerol, butylene glycol or propylene glycol;
- anti-inflammatory agents;
- agents which act on the energy metabolism of cells;
- insect repellents;
- substance P antagonists or CGRP antagonists;
- agents for combating hair loss and/or for restoring the hair;
- anti-wrinkle agents;
- additional inorganic and organic UV-screening agents such as those previously mentioned.

Needless to say, those skilled in the art will take care to select the optional additional compound(s) mentioned above and/or the amounts thereof such that the advantageous properties intrinsically associated with the compositions in accordance with the invention are not, or are not substantially, adversely affected by the envisaged addition(s).

Those skilled in the art will choose said active agent(s) according to the desired effect on the skin, hair, eyelashes, eyebrows or nails.

[Cosmetic Method or Process]

The cosmetic compositions according to the invention have applications in a great number of treatments, in particular cosmetic treatments, of the skin, lips and hair, including the scalp.

Another subject of the present invention is constituted of the use of the compositions according to the invention as defined above in the manufacture of products for the cosmetic treatment of the skin, lips, nails, hair, eyelashes, eyebrows and/or scalp, in particular care products, anti-sun products and make-up products.

The cosmetic compositions according to the invention can be used, for example, as make-up products.

The cosmetic compositions according to the invention may be used, for example, as care products and/or anti-sun protection products for the face and/or the body, of liquid to semi-liquid consistency, such as milks, more or less rich creams, cream-gels and pastes. They may optionally be packaged in aerosol form and may be in the form of a mousse or a spray.

The compositions according to the invention in the form of vaporizable fluid lotions in accordance with the invention are applied to the skin or the hair in the form of fine particles by means of pressurization devices. The devices in accordance with the invention are well known to those skilled in the art and comprise non-aerosol pumps or "atomizers", aerosol containers comprising a propellant and aerosol pumps using compressed air as propellant. These devices are described in patents US 4 077 441 and US 4 850 517.

The compositions packaged as an aerosol in accordance with the invention generally comprise conventional propellants, such as, for example, hydrofluorinated compounds, dichlorodifluoromethane, difluoroethane, dimethyl ether, isobutane, n-butane, propane or trichlorofluoromethane. They are preferably present in amounts ranging from 15% to 50% by weight relative to the total weight of the composition.

The present invention also relates to a cosmetic method, in particular, a non-therapeutic cosmetic method for caring for and/or making up the skin, or for forming a substantially even film on the skin. Each possible non-therapeutic cosmetic method includes applying the cosmetic composition according to the present invention onto the skin.

The cosmetic method according to the present invention can provide cosmetic effects to the skin, such as the skin of the face, by the UV filtering effects provided by the (b)-(ii) lipophilic UV filter(s) in the cosmetic composition used in the present invention. The UV filtering effects can be the basis of caring for and/or making up the skin. The formation of a substantially even or homogeneous film on the skin can provide even or uniform UV filtering effects. The cosmetic effects may include limiting the darkening of the skin, improving the color and uniformity of the complexion, and/or treating aging of the skin.

EXAMPLES

The present invention will be described in a more detailed manner by way of examples. However, these examples should not be construed as limiting the scope of the present invention.

**Example 1 and Comparative Examples 1-3**

[Preparations]

The following cosmetic compositions in the form of an O/W emulsion according to Example 1 and Comparative Examples 1-3, shown in Table 1, were prepared by mixing the components shown in Table 1. The numerical values for the amounts of the components shown in the Tables are all based on "% by weight" as active raw materials.

[Evaluations]

(Film Score)

Each composition according to Example 1 and Comparative Examples 1-3 was applied onto a PP (polypropylene) plate with a thickness of 10 μm, and the evenness of the film was visually evaluated in accordance with the following score system.

1: Very Poor

2: Poor

3: Fair

4: Good

5: Very Good

The average scores for Example 1 and Comparative Examples 1-3 are shown in Table 1.

(In vitro SPF)

Each composition according to Example 1 and Comparative Examples 1-3 was applied onto a PMMA plate in an amount of 1.0 mg/cm2, and the SPF value was measured by a SPF analyzer UV 2000.
The measured in vitro SPF values for Example 1 and Comparative Examples 1-3 are shown in Table 1.

Table 1

| | Example 1 | Comparative Example 1 | Comparative Example 2 | Comparative Example 3 |
|---|---|---|---|---|
| Acrylates/$C_{10-30}$ Alkyl Acrylate Crosspolymer | 0.2 | 0.2 | 0.2 | 0.2 |
| Water | q.s. 100 | q.s. 100 | q.s. 100 | q.s. 100 |
| Ceteth-10 | 0.2 | 0.2 | 0.2 | 0.2 |
| Ethylhexyl Methoxycinnamate | 5.0 | 5.0 | 5.0 | 5.0 |
| Bis(ethylhexyloxyphenyl) Methoxyphenyltriazine | 2.0 | 2.0 | 2.0 | 2.0 |
| KOH | 0.17 | 0.17 | 0.17 | 0.17 |
| Stearic Acid | 1.0 | - | 1.0 | 1.0 |
| Isopropyl Lauroyl Sarcosinate | 5.0 | 5.0 | - | 5.0 |
| Vaseline | - | - | 5.0 | - |

(continued)

| | Example 1 | Comparative Example 1 | Comparative Example 2 | Comparative Example 3 |
|---|---|---|---|---|
| Styrene/Acrylates Copolymer | 2.0 | 2.0 | 2.0 | - |
| Silica | - | - | - | 2.0 |
| Film Score | 5.0 | 4.5 | 2.0 | 1.0 |
| In vitro SPF | 11 | 5 | 4.5 | 1.9 |

Example 1 and Comparative Examples 1-3 are in the form of an O/W emulsion, and they could provide a fresh, water-like texture or good feeling during use, due to the continuous aqueous phase therein.

Example 1 shows that a combination of the polar oil (isopropyl lauroyl sarcosinate), the lipophilic UV filters (ethylhexyl methoxycinnamate and bis(ethylhexyloxyphenyl) methoxyphenyltriazine), the fatty acid (stearic acid), the associative polymer (acrylates/$C_{10}$-$_{30}$ alkyl acrylate crosspolymer), and the hollow particle (styrene/acrylates copolymer) provided both a homogeneous or even film on the skin and high UV-shielding ability.

Comparative Examples 1-3, which lack the fatty acid, the polar oil, or the hollow particle, respectively, could not provide both a homogeneous or even film on the skin and high UV-shielding ability.

Accordingly, only Example 1 could provide both effective UV protection and good feeling during use, among Example 1 and Comparative Examples 1-3.

**Claims**

1. A cosmetic composition in the form of an O/W emulsion, comprising, in a cosmetically acceptable medium:

    (a) at least one aqueous phase comprising water;
    (b) at least one fatty phase comprising,

        (i) at least one polar oil,
        (ii) at least one lipophilic UV filter, and
        (iii) at least one fatty acid;

    (c) at least one anionic associative polymer; and
    (d) at least one hollow particle.

2. The composition according to Claim 1, wherein the amount of water is equal to or more than 55% by weight in relation to the total weight of the composition.

3. The composition according to Claim 1 or 2, wherein the (b)-(i) polar oil is selected from the group consisting of $C_{12}$-$C_{15}$ alcohol benzoate, diisopropyl sebacate, isopropyl lauroyl sarcosinate, dicaprylyl carbonate, 2-phenylethyl benzoate, butyloctyl salicylate, 2-octyldodecyl neopentanoate, dicaprylyl ether, isocetyl stearate, isodecyl neopentanoate, isononyl isononate, isopropyl myristate, isopropyl palmitate, isostearyl behenate, myristyl myristate, octyl palmitate, and tridecyl trimellitate.

4. The composition according to any one Claims 1 to 3, wherein the (b)-(ii) lipophilic UV filter is selected from the group consisting of butylmethoxydibenzoylmethane, ethylhexyl methoxycinnamate, ethylhexyl salicylate, homosalate, butylmethoxydibenzoylmethane, octocrylene, benzophenone-3, n-hexyl 2-(4-diethylamino-2-hydroxybenzoyl)benzoate, 4-methylbenzylidenecamphor, bis(ethylhexyloxyphenyl)methoxyphenyltriazine, ethylhexyl triazone, diethylhexyl butamido triazone, 2,4,6-tris(dineopentyl 4'-aminobenzalmalonate)-s-triazine, 2,4,6-tris(diisobutyl 4'-aminobenzalmalonate)-s-triazine, 2,4-bis(dineopentyl 4'-aminobenzalmalonate)-6-(n-butyl 4'-aminobenzoate)-s-triazine, 2,4,6-tris(biphenyl-4-yl)-1,3,5-triazine, 2,4,6-tris(terphenyl)-1,3,5-triazine, drometrizole trisiloxane, polysilicone-15, 1,1-dicarboxy(2,2'-dimethylpropyl)-4,4-diphenylbutadiene, 2,4-bis [4-[5-(1,1-dimethylpropyl)benzoxazol-2-yl]phenylimino]-6-[(2-ethylhexyl)imino]-1,3,5-triazine, and mixtures thereof.

5. The composition according to any one of Claims 1 to 4, wherein the (b)-(iii) fatty acid is a linear or branched fatty acid having a carbon chain length of $C_{10-30}$.

6. The composition according to any one of Claims 1 to 5, wherein the (c) associative polymer is selected from polymers comprising at least one hydrophilic unit of unsaturated olefinic carboxylic acid type, and at least one hydrophobic unit of the type such as a ($C_{10}$-$C_{30}$) alkyl ester of an unsaturated carboxylic acid.

7. The composition according to any one of Claims 1 to 6, wherein the (d) hollow particle is made from a copolymer of styrene and (meth)acrylic acid or one of its $C_1$-$C_{20}$ alkyl esters.

8. The composition according to claim any one of Claims 1 to 7, wherein the amount of the (b) fatty phase is from 5% to 30% by weight, more preferably from 7% to 25% by weight, and more preferably 10% to 20% by weight in relation to the total weight of the composition.

9. The composition according to any one of Claims 1 to 8, wherein the amount of the (b)-(i) polar oil is from 1% to 15% by weight in relation to the total weight of the composition.

10. The composition according to any one of Claims 1 to 9, wherein the amount of the (b)-(ii) lipophilic UV filter is from 3% to 20% by weight in relation to the total weight of the composition.

11. The composition according to any one of Claims 1 to 10, wherein the amount of the (b)-(iii) fatty acid is from 0.1% to 5% by weight in relation to the total weight of the composition.

12. The composition according to any one of Claims 1 to 11, wherein the amount of the (c) associative polymer is from 0.01% to 5% by weight in relation to the total weight of the composition.

13. The composition according to any one of Claims 1 to 12, wherein the amount of the (d) hollow particle is from 0.1% to 5% by weight in relation to the total weight of the composition.

14. A cosmetic composition according to any one of Claims 1 to 13 for use in a method for caring for and/or making up the skin, comprising applying the composition on the skin.

15. A cosmetic composition according to any one of Claims 1 to 13 for use in a method for forming a substantially even film on the skin, comprising applying the composition on the skin.

**Patentansprüche**

1. Kosmetische Zusammensetzung in Form einer Ö/W-Emulsion, die in einem kosmetisch akzeptablen Medium Folgendes umfasst:

   (a) mindestens eine wässrige Phase, die Wasser umfasst;
   (b) mindestens eine Fettphase umfassend

      (i) mindestens in polares Öl,
      (ii) mindestens einen lipophilen UV-Filter, und
      (iii) mindestens eine Fettsäure;

   (c) mindestens ein anionisches assoziatives Polymer; und
   (d) mindestens ein Hohlteilchen.

2. Zusammensetzung nach Anspruch 1, wobei die Menge an Wasser gleich oder größer ist als 55 Gew.-% bezogen auf das Gesamtgewicht der Zusammensetzung.

3. Zusammensetzung nach Anspruch 1 oder 2, wobei das (b)-(1) polare Öl aus der aus $C_{12}$-$C_{15}$-Alkoholbenzoat, Diisopropylsebacat, Isopropyllauroylsarcosinat, Dicaprylylcarbonat, 2-Phenylethylbenzoat, Butyloctylsalicylat, 2-Octyldodecylneopentanoat, Dicaprylylether, Isocetylstearat, Isodecylneopentanoat, Isononylisononat, Isopropylmyristat, Isopropylpalmitat, Isostearylbehenat, Myristylmyristat, Octylpalmitat und Tridecyltrimellitat bestehenden Gruppe ausgewählt ist.

4. Zusammensetzung nach einem der Ansprüche 1 bis 3, wobei der (b)-(ii) lipophile UV-Filter aus der aus Butylmethoxydibenzoylmethan, Ethylhexylmethoxycinnamat, Ethylhexylsalicylat, Homosalat, Buthylmethoxydibenzoylmethan, Octocrylen, Benzophenon-3, n-Hexyl 2-(4-diethylamino-2-hydroxybenzoyl)benzoat, 4-Methylbenzylidencampher, Bis(ethylhexyloxyphenyl)methoxyphenyltriazin, Ethylhexyltriazon, Diethylhexylbutamidotriazon, 2,4,6-Tris(dineopentyl 4'-aminobenzalmalonat)-s-triazin, 2,4,6-Tris(diisobutyl 4'-aminobenzalmalonat)-s-triazin, 2,4-Bis(dineopentyl 4'-aminobenzalmalonat)-6-(n-butyl 4'-aminobenzoat)-s-triazin, 2,4,6-Tris(biphenyl-4-yl)-1,3,5-triazin, 2,4,6-Tris(terphenyl)-1,3,5-triazin, Drometrizol-Trisiloxan, Polysilicon-15, 1,1-Dicarboxy(2,2'-dimethylpropyl)-4,4-diphenylbutadien, 2,4-Bis[4-[5-(1,1-dimethylpropyl)benzoxazol-2-yl]phenylimino]-6-[(2-ethylhexyl)imino]-1,3,5-triazin und Mischungen davon bestehenden Gruppe ausgewählt ist.

5. Zusammensetzung nach einem der Ansprüche 1 bis 4, wobei die (b)-(iii) Fettsäure eine lineare oder verzweigte Fettsäure mit einer Kohlenstoffkettenlänge von $C_{10-30}$ ist.

6. Zusammensetzung nach einem der Ansprüche 1 bis 5, wobei das (c) assoziative Polymer aus Polymeren ausgewählt ist, die mindestens eine hydrophile Einheit vom Typ einer ungesättigten olefinischen Carbonsäure und mindestens eine hydrophobe Einheit vom Typ wie zum Beispiel einem ($C_{10}$-$C_{30}$)-Alkylester einer ungesättigten Carbonsäure umfassen.

7. Zusammensetzung nach einem der Ansprüche 1 bis 6, wobei das (d) Hohlteilchen aus einem Copolymer von Styrol und (Meth)acrylsäure oder einem ihrer $C_1$-$C_{20}$-Alkylester hergestellt ist.

8. Zusammensetzung nach einem der Ansprüche 1 bis 7, wobei die Menge der (b) Fettphase von 5 Gew.-% bis 30 Gew.-%, mehr bevorzugt von 7 Gew.-% bis 25 Gew.-% und noch mehr bevorzugt von 10 Gew.-% bis 20 Gew.-% bezogen auf das Gesamtgewicht der Zusammensetzung beträgt.

9. Zusammensetzung nach einem der Ansprüche 1 bis 8, wobei die Menge des (b)-(i) polaren Öls von 1 Gew.-% bis 15 Gew.-% bezogen auf das Gesamtgewicht der Zusammensetzung beträgt.

10. Zusammensetzung nach einem der Ansprüche 1 bis 9, wobei die Menge des (b)-(ii) lipophilen UV-Filters von 3 Gew.-% bis 20 Gew.-% bezogen auf das Gesamtgewicht der Zusammensetzung beträgt.

11. Zusammensetzung nach einem der Ansprüche 1 bis 10, wobei die Menge der (b)-(iii) Fettsäure von 0,1 Gew.-% bis 5 Gew.-% bezogen auf das Gesamtgewicht der Zusammensetzung beträgt.

12. Zusammensetzung nach einem der Ansprüche 1 bis 11, wobei die Menge des (c) assoziativen Polymers von 0,01 Gew.-% bis 5 Gew.-% bezogen auf das Gesamtgewicht der Zusammensetzung beträgt.

13. Zusammensetzung nach einem der Ansprüche 1 bis 12, wobei die Menge des (d) Hohlteilchens von 0,1 Gew.-% bis 5 Gew.-% bezogen auf das Gesamtgewicht der Zusammensetzung beträgt.

14. Kosmetische Zusammensetzung nach einem der Ansprüche 1 bis 13 zur Verwendung bei einem Verfahren zum Pflegen und/oder Schminken der Haut, bei dem die Zusammensetzung auf die Haut aufgebracht wird.

15. Kosmetische Zusammensetzung nach einem der Ansprüche 1 bis 13 zur Verwendung bei einem Verfahren zur Bildung eines im Wesentlichen gleichmäßigen Films auf der Haut, bei dem die Zusammensetzung auf die Haut aufgebracht wird.

## Revendications

1. Composition cosmétique sous la forme d'une émulsion H/E (huile dans l'eau), qui comprend, au sein d'un milieu admissible en cosmétologie,

   (a) au moins une phase aqueuse comprenant de l'eau,
   (b) au moins une phase grasse comprenant :

      (i) au moins une huile polaire,
      (ii) au moins un filtre anti-UV lipophile,
      (iii) et au moins un acide gras,

(c) au moins un polymère associatif anionique,
(d) et au moins une particule creuse.

**2.** Composition conforme à la revendication 1, dans laquelle la proportion d'eau, rapportée au poids total de la composition, est égale ou supérieure à 55 % en poids.

**3.** Composition conforme à la revendication 1 ou la revendication 2, dans laquelle l'huile polaire (b)-(i) est choisie dans l'ensemble formé par les composés suivants : benzoate d'alcool en $C_{12}$-$C_{15}$, sébaçate de diisopropyle [nom d'après la nomenclature internationale des ingrédients cosmétiques, INCI: diisopropyl sebacate], *N*-dodécanoyl-*N*-méthylglycinate d'isopropyle [INCI : isopropyl lauroyl sarcosinate], carbonate de dioctyle [INCI : dicaprylyl carbonate], benzoate de 2-phényl-éthyle, salicylate de butyl-octyle, néopentanoate de 2-octyl-dodécyle, oxyde de dioctyle [INCI : dicaprylyl ether], stéarate d'isohexadécyle [INCI : isocetyl stearate], néopentanoate d'isodécyle, isononanoate d'isononyle, myristate d'isopropyle, palmitate d'isopropyle, docosanoate d'isooctadécyle [INCI : isostearyl behenate], myristate de tétradécyle [INCI: myristyl myristate], palmitate d'octyle et 1,2,4-benzène-tricarboxylate de tridécyle [INCI : tridecyl trimellitate].

**4.** Composition conforme à l'une quelconque des revendications 1 à 3, dans laquelle le filtre anti-UV lipophile (b)-(ii) est choisi dans l'ensemble formé par les composés suivants : butyl-méthoxydibenzoylméthane, 4-méthoxycinnamate de 2-éthylhexyle [INCI : ethylhexyl methoxycinnamate], salicylate de 2-éthylhexyle [INCI : ethylhexyl salicylate], salicylate de 3,3,5-triméthyl-cyclohexyle [INCI : homosalate], butyl-méthoxydibenzoylméthane, 2-cyano-3,3-diphényl-acrylate de 2-éthylhexyle [INCI : octocrylene], 2-hydroxy-4-méthoxy-benzophénone [INCI : benzophenone-3], 2-(4-diéthylamino-2-hydroxybenzoyl)-benzoate de n-hexyle, 4-méthylbenzylidène-camphre [INCI : 4-methylbenzylidenecamphor], bis(éthylhexyloxyphényl)-méthoxyphényl-triazine, ethylhexyl triazone [nom INCI], diethylhexyl butamido triazone [nom INCI], 2,4,6-tris(4'-aminobenzalmalonate de dinéopentyle)-s-triazine, 2,4,6-tris(4'-aminobenzalmalonate de diisobutyle)-s-triazine, 2,4-bis(4'-aminobenzalmalonate de dinéopentyle)-6-(4'-aminobenzoate de n-butyle)-s-triazine, 2,4,6-tris(biphényl-4-yl)-1,3,5-triazine, 2,4,6-tris(terphényl)-1,3,5-triazine, drometrizole trisiloxane [nom INCI], polysilicone-15, 1,1-dicarboxy-(2,2'-diméthylpropyl)-4,4-diphénylbutadiène et 2,4-bis[4-[5-(1,1-diméthylpropyl)-benzoxazol-2-yl]-phénylimino]-6-[(2-éthylhexyl)-imino]-1,3,5-triazine, ainsi que leurs mélanges.

**5.** Composition conforme à l'une quelconque des revendications 1 à 4, dans laquelle l'acide gras (b)-(iii) est un acide gras linéaire ou ramifié présentant une longueur de chaîne carbonée de $C_{10}$ à $C_{30}$.

**6.** Composition conforme à l'une quelconque des revendications 1 à 5, dans laquelle le polymère associatif (c) est choisi parmi des polymères comportant au moins un motif hydrophile du type acide carboxylique à insaturation oléfinique, et au moins un motif hydrophobe tel qu'un motif du type ester d'alkyle en $C_{10}$-$C_{30}$ d'un acide carboxylique insaturé.

**7.** Composition conforme à l'une quelconque des revendications 1 à 6, dans laquelle la particule creuse (d) est constituée d'un copolymère de styrène et d'acide (méth)acrylique ou de l'un des esters d'alkyle en $C_1$-$C_{20}$ d'acide (méth)acrylique.

**8.** Composition conforme à l'une quelconque des revendications 1 à 7, dans laquelle la proportion de phase grasse (b), rapportée au poids total de la composition, vaut de 5 % à 30 % en poids, mieux encore de 7 % à 25 % en poids, et encore mieux 10 % à 20 % en poids.

**9.** Composition conforme à l'une quelconque des revendications 1 à 8, dans laquelle la proportion d'huile polaire (b)-(i), rapportée au poids total de la composition, vaut de 1 % à 15 % en poids.

**10.** Composition conforme à l'une quelconque des revendications 1 à 9, dans laquelle la proportion de filtre anti-UV lipophile (b)-(ii), rapportée au poids total de la composition, vaut de 3 % à 20 % en poids.

**11.** Composition conforme à l'une quelconque des revendications 1 à 10, dans laquelle la proportion d'acide gras (b)-(iii), rapportée au poids total de la composition, vaut de 0,1 % à 5 % en poids.

**12.** Composition conforme à l'une quelconque des revendications 1 à 11, dans laquelle la proportion de polymère associatif (c), rapportée au poids total de la composition, vaut de 0,01 % à 5 % en poids.

**13.** Composition conforme à l'une quelconque des revendications 1 à 12, dans laquelle la proportion de particule creuse

(d), rapportée au poids total de la composition, vaut de 0,1 % à 5 % en poids.

**14.** Composition cosmétique conforme à l'une quelconque des revendications 1 à 13 pour l'utilisation dans un procédé de soin et/ou de maquillage de la peau, comprenant le fait d'appliquer la composition sur la peau.

**15.** Composition cosmétique conforme à l'une quelconque des revendications 1 à 13 pour l'utilisation dans un procédé de formation d'un film pratiquement uni sur la peau, comprenant le fait d'appliquer la composition sur la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050031655 A1
- US 6495123 B1
- WO 2011042358 A1
- FR 0853634
- EP 0955039 A
- US 2004175338 A
- US 5624663 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- WO 04006878 A
- WO 05058269 A
- WO 06032741 A
- FR 2957249
- FR 2957250
- EP 0841341 A
- EP 0216479 A
- US 3915921 A
- US 4509949 A
- EP 0173109 A
- FR 0009609
- WO 9844012 A
- US 4427836 A
- US 4469825 A
- US 4594363 A
- US 4677003 A
- US 4920160 A
- US 4970241 A
- EP 267726 A
- EP 331421 A
- US 490229 A
- US 5157084 A
- US 5663213 A
- EP 1092421 A
- WO 9206778 A
- EP 1069142 A
- FR 2315991
- FR 2416008
- US 4077441 A
- US 4850517 A


### Non-patent literature cited in the description


- *Personal Care SUNSPHERES™ Hollow Sphere Technology An SPF Booster for More Aesthetically Pleasing Formulations Features, Benefits and Applications, http:// www.dow.com/assets/attachments/business/pcare/sunspheres/sunspheres_powder/tds/sunspheres_powder.pdf*
- **C.M. HANSEN.** The three dimensional solubility parameters. *J. Paint Technol.,* 1967, vol. 39, 105
- **G. FONNUM ; J. BAKKE ; FK. HANSEN.** *Colloid Polym. Sci,* 1993, vol. 271, 380-389
- **BANGHAM ; STANDISH ; WATKINS.** *J. Mol. Biol.,* 1965, vol. 13, 238